# EUROPEAN PATENT APPLICATION

(11) **EP 1 842 499 A1**
(43) Date of publication of application: **10.10.2007**
(21) Application number: 06711971.9
(22) Date of filing: 19.01.2006
(51) Int. Cl.: A61B 17/28, A61B 1/00, A61M 1/00

(54) **TREATMENT INSTRUMENT FOR ENDOSCOPE**

(30) Priority: 27.01.2005 JP 2005019346
(71) Applicant: Olympus Corporation, Tokyo 151-0072 (JP)
(72) Inventor: NAKAGAWA, Tsuyoshi, Tokyo 1890025 (JP); YANUMA, Yutaka, Tokyo 1860004 (JP); INOUE, Yoshimitsu, Tokyo 1910001 (JP); SUYAMA, Makoto, Aomori, 0360351 (JP); IWABUCHI, Akihisa, Higashimatsuyama-sama, 3550018 (JP)
(74) Representative: von Hellfeld, Axel
(86) International application number: PCT/JP2006/300725
(87) International publication number: WO 2006/080231

(57) **Abstract**

A drainage tube retainer 1 as an endoscopic treatment instrument includes a guide catheter 10 for slidably supporting a drainage tube 6 retained in a live organ through a channel; a pusher tube 11 disposed in the exterior of the guide catheter 10 and slidably supported by the guide catheter 10; and a pusher cap 13 provided to the pusher tube 11 for positioning the pusher tube 11 relative to the endoscope so that a direction in which the pusher tube 11 is inserted through the channel substantially coincides with a direction in which the guide catheter 10 is retracted from the pusher tube 11.

## Description

### Technical Field

The present invention relates to an endoscopic treatment instrument used for endoscopic surgery and endoscopy for pancreaticobiliary ducts.
The present application is based on patent application No. 2005-0019346 filed January 27, 2005, in Japan, the content of which is incorporated herein by reference.

### Background Art

A drainage tube (drainage tube) is retained in a human body through an endoscope inserted into the human body during a manipulation conducted for secretory fluid drainage from a bile duct and a pancreatic duct.
In order to retain the drainage tube in the bile duct or the pancreatic duct through a channel of the endoscope, the drainage tube retainer proposed as an endoscopic treatment instrument includes a guide tube that can be extended and retracted along an elastic guidewire previously inserted in the human body; and an elastic pusher tube for pushing the drainage tube fit to the guide tube toward a distal end of the guide tube (see Patent documents 1 and 2 as follows).

First, to retain the drainage tube in the bile duct with the foregoing drainage tube retainer, the distal end of the guidewire is inserted through a treatment instrument insertion channel formed in the endoscope. The distal end of the guide tube is inserted next into the bile duct along the guidewire inserted through the treatment instrument insertion channel. By fitting the guide tube to the drainage tube and pushing the drainage tube at the distal end of the pusher tube while guiding with the guide tube, the drainage tube moves into the bile duct, thereby causing the drainage tube to protrude above the guidewire. The pusher tube, the guide tube, and the guidewire are retracted; thus, the drainage tube is eventually retained in the bile duct.

The guide tube of the foregoing conventional endoscopic treatment instrument must be maintained at a certain fixed position relative to the endoscope. However, an attempt to slide the pusher tube along the guide tube moves the guide tube together with the pusher tube because of a frictional force between the guide tube and the pusher tube; therefore, the position of the guide tube inevitably changes relative to the endoscope. In order to avoid the positional change of the guide tube relative to the endoscope, the movement of the pusher tube toward a distal end of the guide tube must be carried out simultaneously by the same amount as the movement of the guide tube toward a manipulation side. Since these movements cannot be carried out by the endoscopist alone, the endoscopist and a supporter will meet difficulty due to such a complex cooperation.
Patent document 1: Japanese Unexamined Patent Application, First Publication No. H11-76412
Patent Document 2: Japanese Unexamined Patent Application, First Publication No. 2000-152985

### DISCLOSURE OF INVENTION

### Problems to be solved by the Invention

The present invention was conceived in consideration of the foregoing circumstances, and an object thereof is to provide an endoscopic treatment instrument with which an endoscopist alone can slide a hollow section along an elongated section that is inserted in the hollow section such that the elongated section is retained at a certain fixed position.

### MEANS FOR SOLVING THE PROBLEMS

An endoscopic treatment instrument according to the present invention inserted through a channel of an endoscope from a endoscope port includes: an elongated section for slidably supporting a retainer retained in a live organ through the channel; a hollow section disposed in the exterior of the elongated section and slidably supported by the elongated section; and a connecting section provided to the hollow section for positioning the hollow section relative to the endoscope so that a direction in which the hollow section is inserted through the channel substantially coincides with a direction in which the hollow section is retracted from the hollow section.

Since the endoscopic treatment instrument positions the hollow section relative to the endoscope via the connecting section, the direction in which the hollow section is inserted through the channel of the endoscope substantially coincides with the direction in which the elongated section is retracted from the channel; therefore the operations to insert the hollow section through the channel and to retract the elongated section from the hollow section can be carried out simultaneously by one-handed manipulation. Carrying out these two operations simultaneously permits the hollow section to be inserted through the channel. On the other hand, the elongated section is retracted from the hollow section by the length equal to an insertion length of the hollow section. To be more specific, apparently the elongated section remains at a predetermined position in the channel and only the hollow section is inserted through the channel along the elongated section. Since the present invention allows an endoscopist alone to carry out the above operations, complex manipulation can be avoided.

In the endoscopic treatment instrument according to the present invention, it is preferable that the elongated section be capable of sliding along a guidewire inserted through the channel.

The endoscopic treatment instrument according to the present invention allows the direction in which the elongated section previously inserted through the endoscope port is inserted through the channel along the guidewire to coincide with the direction in which the guidewire is retracted from the elongated section after the elongated section is fitted to the guidewire so that the guidewire previously inserted through the channel is inserted in the interior of the elongated section; therefore, the operations to insert the elongated section through the channel and to retract the guidewire from the elongated section can be carried simultaneously one-handed. Carrying out these two operations simultaneously permits the elongated section to be inserted through the channel. On the other hand, the guidewire is retracted from the hollow section by the length equal to an insertion length of the elongated section. To be more specific, apparently the guidewire remains at a predetermined position in the channel and only the elongated section is inserted through the channel along the guidewire. Since the present invention allows an endoscopist alone to carry out the above operations, complex manipulation can be avoided.

In the endoscopic treatment instrument according to the present invention, it is preferable that the connecting section be provided with an elastically deformable, approximately C-shaped engagement member.

Since the round surface of the operation section, etc., of the endoscope permits the connecting section to be positioned and engaged at a certain position of the endoscope by elastically deforming the approximately C-shaped engagement member of the endoscopic treatment instrument according to the present invention. In addition, the connecting section can be rotated relative to the endoscope in a direction along an arch of the substantially C-shaped engagement member.

In the endoscopic treatment instrument according to the present invention, it is preferable that the connecting section attached to the endoscope via an endoscope adapter attached to an operation section of the endoscope for rotatively supporting the connecting section be detachable.

The endoscopic treatment instrument according to the present invention allows the proximal end of the hollow section to be fixed in a direction toward the endoscope port separately by a predetermined distance from the operation section of the endoscope by attaching the endoscope adapter to the operation section of the endoscope and attaching the connecting section to the adapter.

It is preferable that the endoscopic treatment instrument according to the present invention further include a cap disposed at a proximal end of the elongated section so that the cap is detachable from the connecting section.

The endoscopic treatment instrument according to the present invention allows the elongated section and the hollow section to be simultaneously inserted through the channel of the endoscope along the guidewire if the cap is previously attached to the connecting section. Also, detaching the cap from the connecting section permits the hollow section to be moved longitudinally along the elongated section.

It is preferable that the endoscopic treatment instrument according to the present invention further include a cap disposed at a proximal end of the elongated section so that the cap is detachable from the elongated section.

The endoscopic treatment instrument according to the present invention allows the hollow section to be fitted to the elongated section not only from the distal end but also from the proximal end if the cap is detached from the elongated section.

In the endoscopic treatment instrument according to the present invention, it is preferable that the cap be provided with an elastically deformable, approximately C-shaped engagement member.

The round surface of the operation section, etc., of the endoscope permits the cap to be positioned and engaged at a certain position of the endoscope by elastically deforming the approximately C-shaped engagement member of the endoscopic treatment instrument according to the present invention. In addition, the cap can be rotated relative to the endoscope in a direction along an arch of the substantially C-shaped engagement member.

In the endoscopic treatment instrument according to the present invention, it is preferable that the cap attached to the endoscope via an endoscope adapter attached to an operation section of the endoscope so as to rotatively support the cap be detachable.

The endoscopic treatment instrument according to the present invention allows the proximal end of the elongated section to be fixed in a direction toward the endoscope port separately by a predetermined distance from the operation section of the endoscope by attaching the endoscope adapter to the operation section of the endoscope and attaching the cap to the adapter.

### Effects of The Invention

The endoscopic treatment instrument according to the present invention allows an endoscopist alone to slide a hollow section along an elongated section so that the elongated section that is inserted in the hollow section is retained at a certain position.

### BRIEF DESCRIPTION OF DRAWINGS

FIG. 1 is a plan view illustrating a drainage tube retainer according to a first embodiment of the present invention.
FIG. 2 is a cross-section illustrating a drainage tube retainer according to the first embodiment of the present invention in detail.
FIG. 3 is a plan view illustrating a drainage tube retained in a human body by the drainage tube retainer according to the first embodiment of the present invention.
FIG. 4 is a schematic diagram illustrating how to use the drainage tube retainer according to the first embodiment of the present invention.
FIG. 5 is a schematic diagram illustrating how to use the drainage tube retainer according to the first embodiment of the present invention.
FIG. 6 is a schematic diagram illustrating how to use the drainage tube retainer according to the first embodiment of the present invention.
FIG. 7 is a schematic diagram illustrating how to use the drainage tube retainer according to the first embodiment of the present invention.
FIG. 8 is a schematic diagram illustrating how to use the drainage tube retainer according to the first embodiment of the present invention.
FIG. 9 is a plan view illustrating a guide cathether constituting the drainage tube retainer according to a second embodiment of the present invention.
FIG. 10 is a cross-section illustrating the guide cathether constituting the drainage tube retainer according to the second embodiment of the present invention.
FIG. 11 is a plan view illustrating a pusher tube constituting the drainage tube retainer according to a second embodiment of the present invention.
FIG. 12 is a schematic diagram illustrating how to use the drainage tube retainer according to the second embodiment of the present invention.
FIG. 13 is a schematic diagram illustrating how to use the drainage tube retainer according to the second embodiment of the present invention.
FIG. 14 is a schematic diagram illustrating how to use the drainage tube retainer according to the second embodiment of the present invention.
FIG. 15 is a schematic diagram illustrating how to use the drainage tube retainer according to the second embodiment of the present invention.
FIG. 16 is a schematic diagram illustrating how to use the drainage tube retainer according to the second embodiment of the present invention.
FIG. 17 is a schematic diagram illustrating how to use the drainage tube retainer according to the second embodiment of the present invention.
FIG. 18 is a schematic diagram illustrating how to use the drainage tube retainer according to the second embodiment of the present invention.
FIG. 19 illustrates a first modified example of the drainage tube.
FIG. 20 is a schematic diagram for illustrating how to use the drainage tube shown in FIG. 19.
FIG. 21 illustrates a second modified example of the drainage tube.
FIG. 22 is a schematic diagram for illustrating how to use the drainage tube shown in FIG. 21.
FIG. 23 is a schematic diagram for illustrating an inappropriate usage of the drainage tube.
FIG. 24 is a schematic diagram for illustrating an appropriate usage of the drainage tube shown in FIG. 21.

### EXPLANATION OF REFERENCE NUMERALS AND SYMBOLS

- 1, 30:: drainage tube retainer (endoscopic treatment instrument)
- 2:: endoscope
- 3:: endoscope port
- 5:: guidewire
- 10, 32:: guide cathether (elongated section)
- 11:: pusher tube (hollow section)
- 13, 37:: pusher cap (connecting section)
- 15, 31:: cap
- 16:: hook (engagement member)
- 22:: endoscope adapter

### BEST MODE FOR CARRYING OUT THE INVENTION

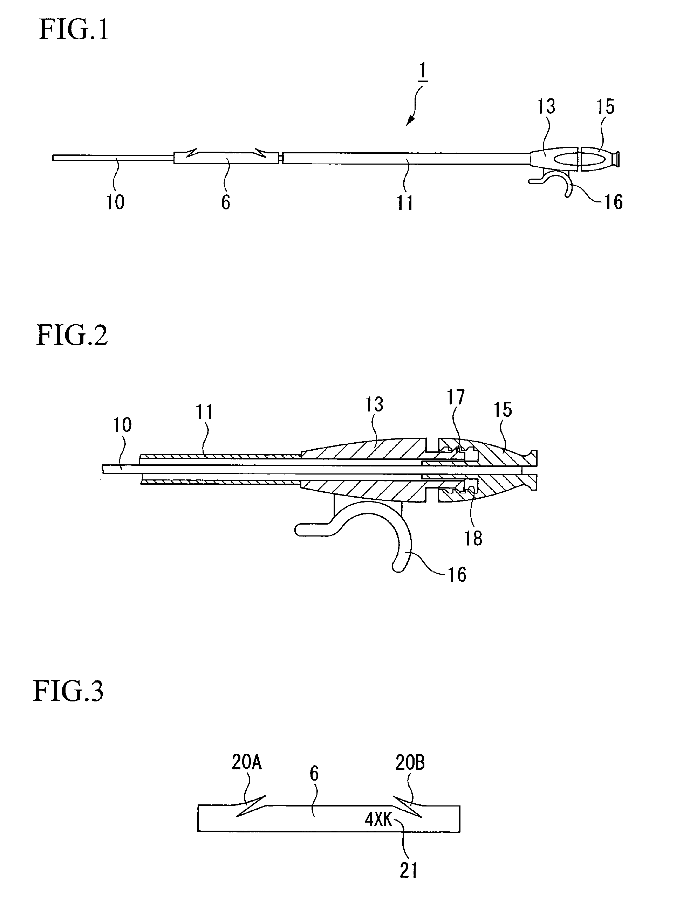

A first embodiment of the present invention will be explained with reference to FIGS. 1 to 8.
As illustrated in FIGS. 1 to 7, a drainage tube retainer (endoscopic treatment instrument) 1 according to the present embodiment is an instrument retained in a constriction 8 in a bile duct 7, etc. The drainage tube retainer 1 can extend and retract in a channel not shown in the drawings along a guidewire 5 inserted in the channel from an endoscope port 3 of an endoscope 2. Provided to the drainage tube retainer 1 are a guide cathether (elongated section) 10; a pusher tube (elongated section) 11; a pusher cap (connecting section) 13; and a cap 15.

The guide catheter 10 fitted to the drainage tube 6 so as to be inserted in the drainage tube 6 slidably supports the drainage tube 6. The guidewire 5 is inserted through the guide catheter 10.
The pusher tube 11 fitted to the guide catheter 10 so as to pass the guide catheter 10 therethrough is slidably supported by the guide catheter 10.

The pusher cap 13 provided to a proximal end of the pusher tube 11 positions the proximal end of the pusher tube 11 relative to an operation section 12 of the endoscope 2 so that a direction in which the pusher tube 11 is inserted into the channel substantially coincides with a direction in which the guide catheter 10 is retracted from the pusher tube 11.
The cap 15 is provided to a proximal end of the guide catheter 10.

The guide catheter 10 is considerably longer than the total length of the pusher tube 11 and the drainage tube 6 so that the distal end of the guide catheter 10 protrudes ahead of the drainage tube 6 when the guide catheter 10 is fitted to the pusher tube 11 and further fitted to the drainage tube 6 disposed ahead of the distal end of the pusher tube 11.
Provided to the pusher cap 13 is an approximately C-shaped and elastically deformable hook (engagement member) 16. The hook 16 is formed to have its distal end directed toward the distal end of the pusher tube 11.
Provided to the proximal end of the pusher cap 13 is a male thread section 17. Detachably provided to the distal end of the cap 15 is a female thread section 18 that engages with the male thread section 17.

As illustrated in FIG. 3, disposed to both ends of the drainage tube 6 are flaps 20A and 20B. The flaps 20A and 20B expand when retained in the interior of the bile duct 7 and engage to living body organs so as to prevent the drainage tube 6 from misaligning.
A mark 21 indicating a manufacturing lot number or a model number, etc., of the drainage tube 6 is printed in the vicinity of one of the flaps 20A and 20B, e.g., the flap 20B, that is disposed nearer to the endoscope 2 when the drainage tube 6 is retained in the body ("4XK" is printed in the example illustrated in FIG. 3.).

Detachably attached to the operation section 12 of the endoscope 2 is an endoscopic adapter 22 that rotatively supports the hook 16 of the pusher cap 13. The endoscopic adapter 22 is provided with an adapter body 23, a cylindrical treatment instrument fixture 25, and an endoscope fixture 26. A hook 16 slidably engages to the treatment instrument fixture 25 provided to an end of the adapter body 23. Provided to the other end of the adapter body 23 is the approximately cylindrical endoscope fixture 26 in which a part of the cylindrical fixture is notched.

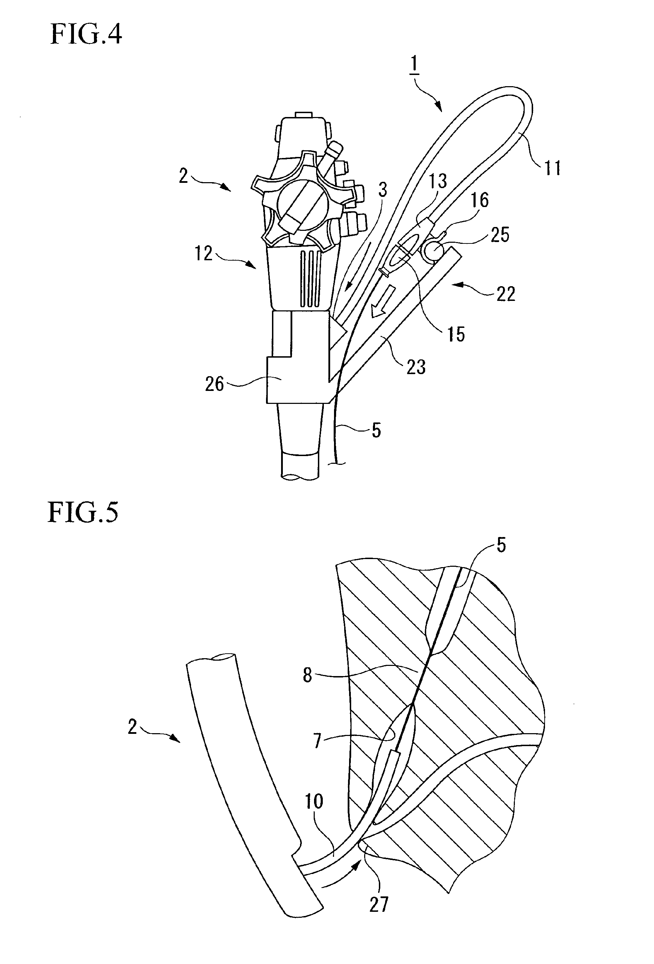

A method, function, and effect according to the present embodiment will be explained next regarding the retaining of the drainage tube 6 with the drainage tube retainer 1.
As illustrated in FIG. 4, an insertion section of the endoscope 2 is first inserted into a body cavity so as to reach the distal end into the vicinity of a duodenal papilla. Consequently the guidewire 5 is inserted into a channel not shown in the drawing from the endoscope port 3 of the endoscope 2 so as to be inserted through the constriction 8 of the bile duct 7.

Consequently, the endoscope fixture 26 of the endoscopic adapter 22 is attached at a predetermined position of the operation section 12 of the endoscope 2. The distal end of the guide catheter 10 fitted to the drainage tube 6 and the pusher tube 11 is fitted to the guidewire 5 so that the proximal end of the guidewire 5 protruding from the endoscope port 3 is inserted in the guide catheter 10. Then a part of the drainage tube 6 and a part of the pusher tube 11 are inserted through the channel.
As illustrated in FIG. 4, the guidewire 5, the guide catheter 10 fitted to the guidewire 5 , and the pusher tube 11 fitted to the guide catheter 10 are folded together at some midpoints thereof them in the vicinity of the endoscope port 3. The hook 16 is then hung on the treatment instrument fixture 25 of the endoscopic adapter 22. Accordingly the hook 16 is adjustably rotated on the treatment instrument fixture 25 so that the cap 15 faces the endoscope port 3 and so that the pusher tube 11 and the guidewire 5 having protruded from the cap 15 are disposed substantially in parallel. Since a direction in which the pusher tube 11 and the guide catheter 10 fitted to the pusher tube 11 are inserted into the channel from the endoscope port 3 substantially coincides with a direction in which the guidewire 5 is retracted from the guide catheter 10, the endoscopist can conduct an operation of inserting the pusher tube 11 and the guide catheter 10 into the channel from the endoscope port 3 and an operation of retracting the guidewire 5 from the guide catheter 10 simultaneously by a single-handed manipulation.

Grasping with the endoscopist's hand and moving together this state of the pusher tube 11, the guide catheter 10, and the guidewire 5 in a direction indicated by an arrow illustrated in the drawing permit the pusher tube 11 and the guide catheter 10 to be inserted through the channel. On the other hand, the guidewire 5 is retracted from the guide catheter 10 by the length equal to an insertion length of the pusher tube 11 and the guide catheter 10. To be more specific, apparently the guidewire 5 remains at a predetermined position in the channel, and only the pusher tube 11 and the guide catheter 10 are inserted through the channel along the guidewire 5. As illustrated in FIG. 5, repeating the above operations permits the pusher tube 11 and the guide catheter 10 to be inserted through the channel while the distal end of the guidewire 5 remains at the predetermined position and permits the distal end of the guide catheter 10 to move to a desirable position.

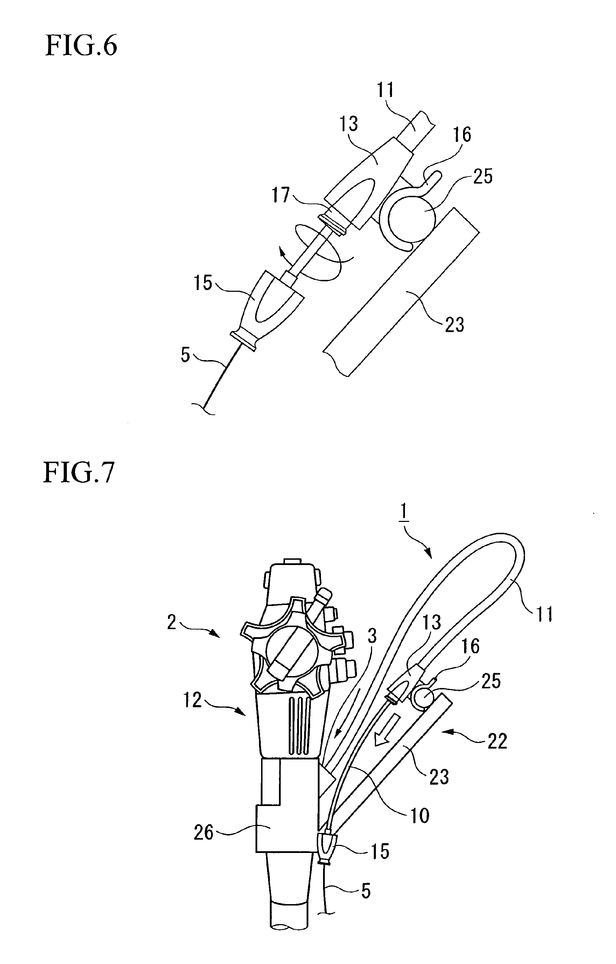

As illustrated in FIG. 6, the cap 15 is rotated relative to the pusher cap 13 so as to remove the cap 15 from the pusher cap 13.
As illustrated in FIG. 7, the cap 15 is grasped and the guide catheter 10 and the guidewire 5 fitted to the guide catheter 10 are retracted from the pusher cap 13 so that the guide catheter 10 and the pusher tube 11 that have protruded from the pusher cap 13 are substantially parallel in the vicinity of the endoscope port 3. Since a direction in which the pusher tube 11 is inserted into the channel from the endoscope port 3 substantially coincides with a direction in which the guide catheter 10 and the guidewire 5 are retracted from the guide catheter 11, the endoscopist can conduct an operation of inserting the pusher tube 11 into the channel from the endoscope port 3 and an operation of retracting the guide catheter 10 and the guidewire 5 from the pusher tube 11 simultaneously by a single-handed manipulation.

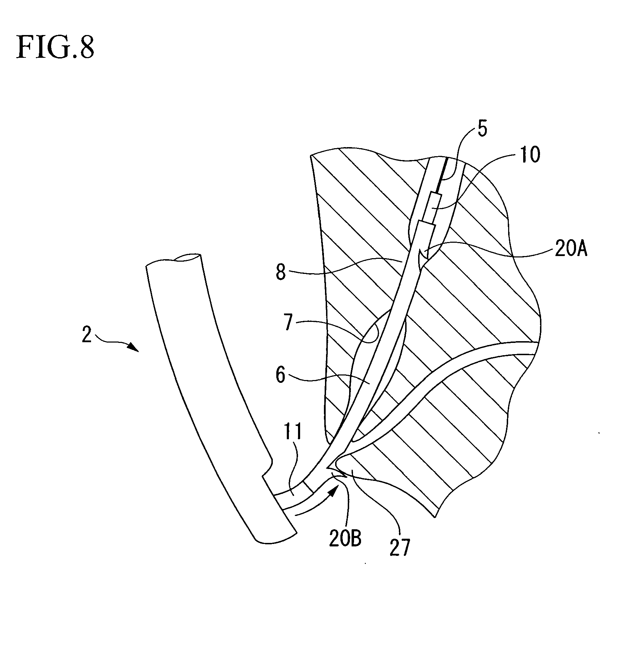

Grasping with the endoscopist's hand and moving together this state of the guide catheter 10, the guidewire 5, and the pusher tube 11 in a direction indicated by an arrow illustrated in the drawing permit the pusher tube 11 to be inserted through the channel.
On the other hand, the guide catheter 10 and the guidewire 5 are retracted from the pusher tube 11 by the length equal to an insertion length of the pusher tube 11. To be more specific, apparently the guide catheter 10 and the guidewire 5 remain at predetermined positions in the channel, and only the pusher tube 11 is inserted through the channel along the guide catheter 10. As illustrated in FIG. 8, repeating the above operations permits the pusher tube 11 to be inserted through the channel while the distal end of the guide catheter 10 and the distal end of the guidewire 5 remain at the predetermined positions, and permits the distal end of the pusher tube 11 to move to a desirable position. The drainage tube 11 is pushed toward the distal end of the pusher tube 11 to be inserted through the bile duct 7.

Retracting the guide catheter 10, the pusher tube 11, and the guidewire 5 from the bile duct 7 and detaching them from the endoscope 2 eventually allow the drainage tube 6 to be retained in the bile duct 7.

The endoscopic adapter 22 attached to the operation section 12 of the endoscope 2 permits the proximal end of the pusher tube 11 of the above drainage tube retainer 1 to be fixed separately from the operation section 12 of the endoscope 2 by a predetermined distance and to be directed toward the endoscope port 3.

In addition, the pusher cap 13 having the elastically deformable hook 16 can be positioned and engaged at a round surface part of the treatment instrument fixture 25. Furthermore, the pusher cap 13 can be rotated relative to the endoscope 2 in a direction along an arch of the substantially C-shaped hook 16.
The guidewire 5, the guide catheter 10, and the pusher tube 11 can be folded in the vicinity of the endoscope port 3; therefore, the guidewire 5 can be disposed substantially in parallel with the guide catheter 10 and the pusher tube 11. This allows the direction in which the pusher tube 11 and the guide catheter 10 are inserted through the channel from the endoscope port 3 to substantially coincide with the direction in which the guidewire 5 is retracted from the guide catheter 10. This furthermore allows the direction in which the pusher tube 11 is inserted through the channel from the endoscope port 3 to substantially coincide with the direction in which the guide catheter 10 and the guidewire 5 are retracted from the pusher tube 11.

This results in allowing the endoscopist alone having connected the cap 15 to the pusher cap 13 to conduct the operation of inserting two tubes, i.e., the guide catheter 10 and the pusher tube 11 fitted to the guide catheter 10 into the channel along the guidewire 5 while the distal end of the guidewire 5 is retained at a certain position. Also, the endoscopist alone having detached the cap 15 from the pusher cap 13 can conduct the operation of inserting the drainage tube 6 and the pusher tube 11 through the channel along the guidewire 5 and the guide catheter 10 while the distal end of the guidewire 5 and the distal end of the guide catheter 10 are retained at certain positions.

Also, the mark 21 indicating a manufacturing lot number, etc., printed not on a package but directly on the drainage tube 6 allows information including the manufacturing lot numbers, etc. of the drainage tube 6 that has been retained in a body.

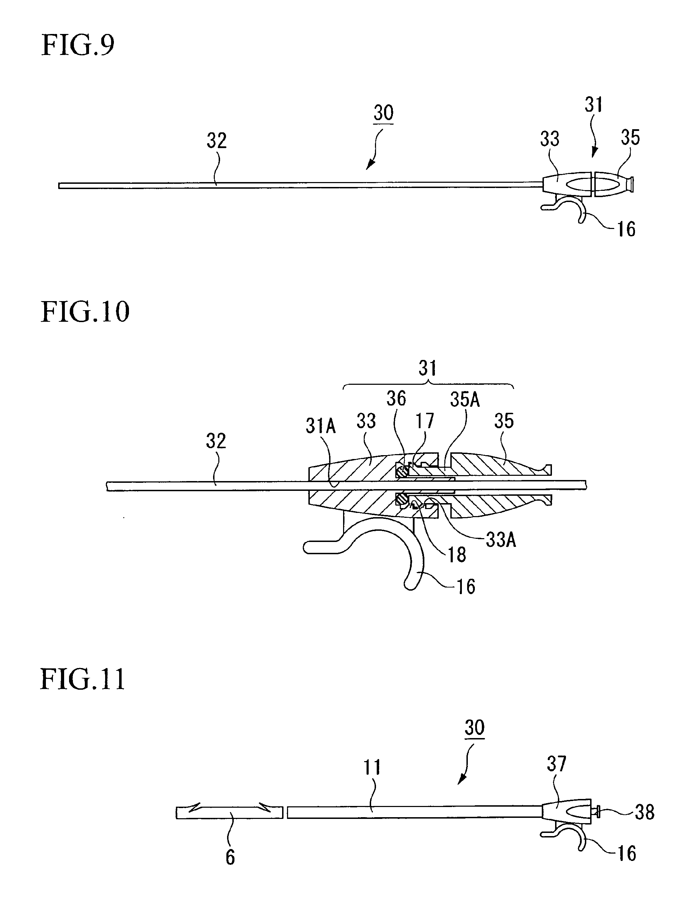

A second embodiment of the present invention will be explained next with reference to FIGS. 9 to 18. Note that elements that are equivalent to those of the above first embodiment will be assigned the same reference symbols, and redundant explanations thereof will be omitted.
Provided to a drainage tube retainer 30 according to the present embodiment is a cap 31 detachable from a guide catheter 32. Provided to the cap 31 are a hub 33, a plunger 35, and an O-ring 36. Provided to the hub 33 is a through-hole 31A through which a guide catheter 32 can penetrate. Also, provided to the hub 33 is a hook 16. Provided to a proximal end of the hub 33 is a screw hole 33A. The inner diameter of the screw hole 33A is greater than that of the through-hole 31A. A female thread section 18 is formed on the surface of the screw hole 33A. Provided to a distal end of the plunger 35 is a protruding screw section 35A. A male thread section 17 that engages with the female thread section 18 of the screw hole 33A is formed on an outer surface of the protruding screw section 35A that protrudes from the plunger 35 toward the screw hole 33A of the hub 33.

The size of the O-ring 36, i.e., the outer diameter thereof enables it to be inserted in the screw hole 33A. The O-ring 36 crushed between a bottom section of the screw hole 33A in the hub 33 and a distal end of the protruding screw section 35A elastically deforms so as to engage with the outer surface of the guide catheter 32. A flange section 38 is provided to the proximal end of the pusher cap 37 in place of the male thread section 17.

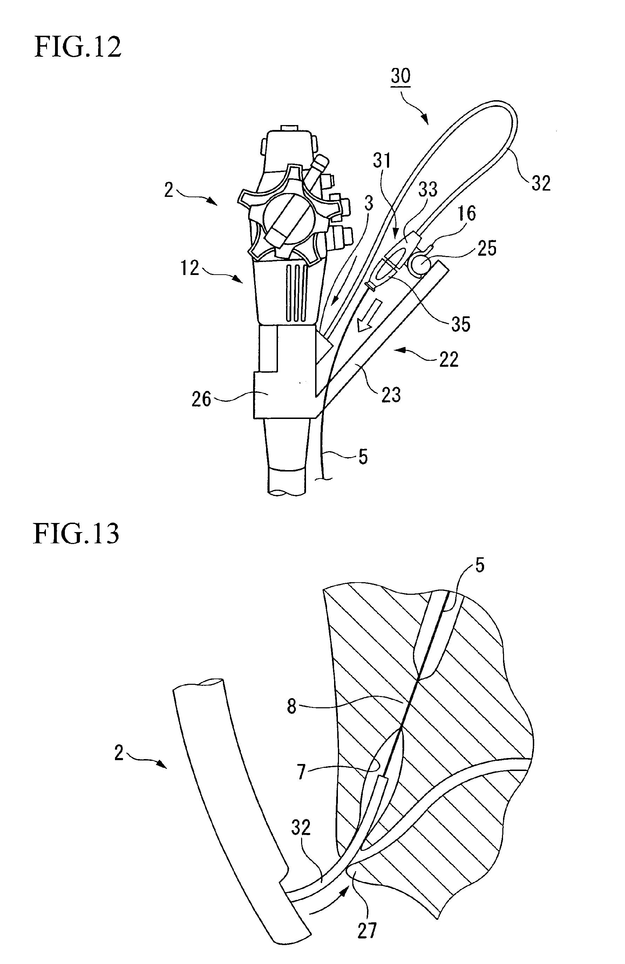

A method, function, and effect according to the present embodiment will be explained next regarding the retaining of the drainage tube 6 with the drainage tube retainer 30.
To begin with, as illustrated in FIG. 13, an insertion section of the endoscope 2 is inserted into a body cavity so as to reach the distal end into the vicinity of a duodenal papilla. Consequently the guidewire 5 is inserted into a channel not shown in the drawing from a endoscope port 3 of the endoscope 2 so as to be inserted through a constriction 8 of a bile duct 7.

Consequently, the endoscope fixture 26 of the endoscopic adapter 22 is attached at a predetermined position of the operation section 12 of the endoscope 2. The distal end of the guide catheter 32 is fitted to the guidewire 5 so that the proximal end of the guidewire 5 having protruded from the endoscope port 3 is inserted in the guide catheter 32; thus, a part of the guide catheter 32 is consequently inserted in the channel.
As illustrated in FIG. 12, the guidewire 5 and the guide catheter 32 fitted to the guidewire 5 are folded together at some midpoints thereof in the vicinity of the endoscope port 3. The hook 16 of the cap 31 is then hung on the treatment instrument fixture 25 of the endoscopic adapter 22. Accordingly, the hook 16 is adjustably rotated on the treatment instrument fixture 25 so that the cap 31 faces the endoscope port 3 and so that the guide catheter 32 and the guidewire 5 having protruded from the cap 31 are disposed substantially in parallel. Since a direction in which the guide catheter 32 is inserted into the channel from the endoscope port 3 substantially coincides with a direction in which the guidewire 5 is retracted from the guide catheter 32, the endoscopist can conduct an operation of inserting the guide catheter 32 into the channel from the endoscope port 3 and an operation of retracting the guidewire 5 from the guide catheter 32 simultaneously by a single-handed manipulation.

Grasping with the endoscopist's hand and moving together this state of the guide catheter 32 and the guidewire 5 in a direction indicated by an arrow illustrated in the drawing permit the guide catheter 32 to be inserted through the channel. On the other hand, the guidewire 5 is retracted from the guide catheter 32 by the length equal to an insertion length of the guide catheter 32. To be more specific, apparently the guidewire 5 remains at a predetermined position in the channel, and only the pusher tube 32 is inserted through the channel along the guidewire 5. As illustrated in FIG. 13, repeating the above operations permits the guide catheter 32 to be inserted through the channel while the distal end of the guidewire 5 remains at the predetermined position, and permits the distal end of the guide catheter 32 to move to a desirable position.

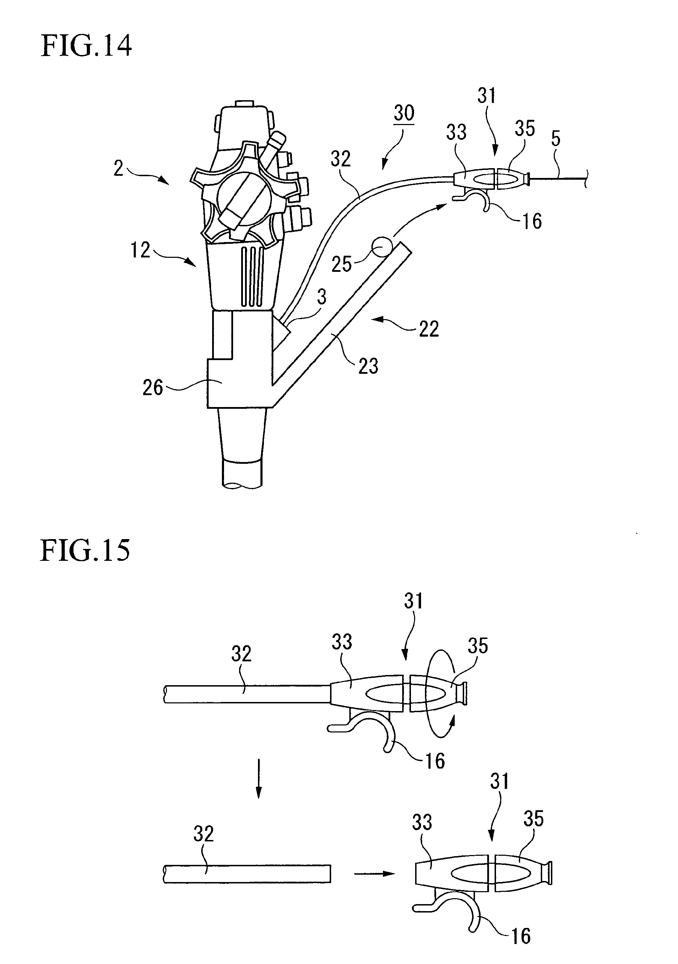

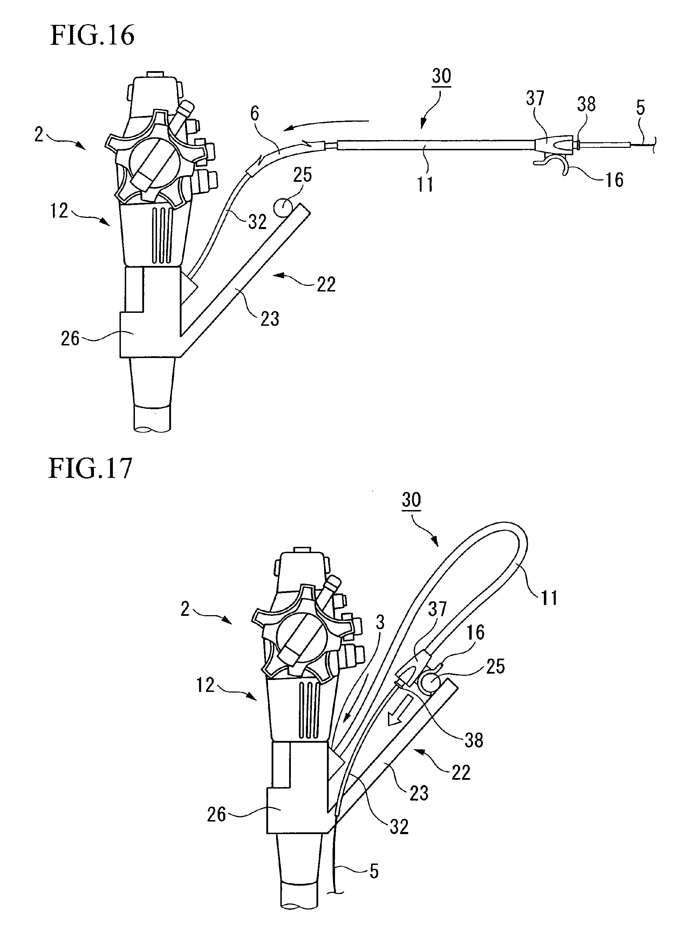

Consequently, the hook 16 of the cap 31 is detached from the treatment instrument fixture 25 of the endoscopic adapter 22 as illustrated in FIG. 16. The plunger 35 is then separated from the hub 33 by rotating the plunger 35 relative to the hub 33 as illustrated in FIG. 15. Separating the plunger 35 from the hub 33 releases the engagement between the guide catheter 32 and the O-ring 36; thus, the cap 31 is detached from the guide catheter 32.

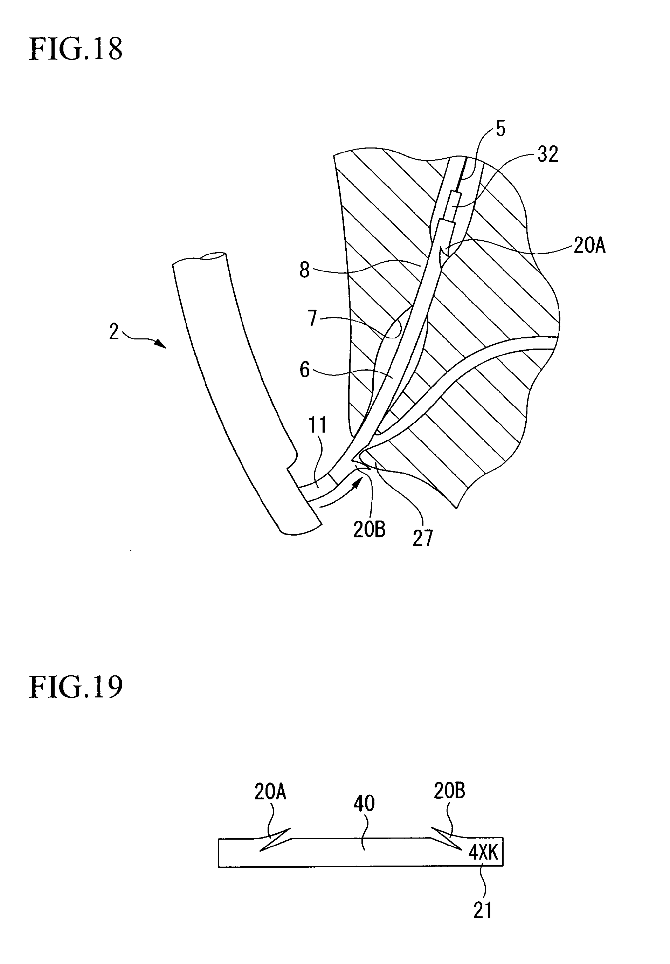

Consequently the proximal end of the guide catheter 32 is inserted through the drainage tube 6 and further inserted through the pusher tube 11 as illustrated in FIG. 16. The guide catheter 32 and the pusher tube 11 are folded together at some midpoints thereof in the vicinity of the endoscope port 3. The hook 16 of the pusher cap 37 is then hung on the treatment instrument fixture 25 of the endoscopic adapter 22. Accordingly the hook 16 is adjustably rotated on the treatment instrument fixture 25 so that, as illustrated in FIG. 17, the cap pusher cap 37 faces the endoscope port 3 and so that the pusher tube 11 and the guide catheter 32 having protruded from the pusher cap 37 are disposed substantially in parallel. Since a direction in which the pusher tube 11 is inserted into the channel from the endoscope port 3 substantially coincides with a direction in which the guide catheter 32 is retracted from the pusher tube 11, the endoscopist can conduct an operation of inserting the pusher tube 11 into the channel from the endoscope port 3 and an operation of retracting the guide catheter 32 from the pusher tube 11 simultaneously by a single-handed manipulation.

Grasping with the endoscopist's hand and moving together this state of the 9 and the guide catheter 32 in a direction indicated by an arrow illustrated in the drawing permit the pusher tube 11 to be inserted through the channel. On the other hand, the guide catheter 32 is retracted from the pusher tube 11 by the length equal to an insertion length of the pusher tube 11. To be more specific, apparently the guide catheter 32 remains at a predetermined position in the channel, and only the pusher tube 11 is inserted through the channel along the guide catheter 32. As illustrated in FIG. 18, repeating the above operations permits the pusher tube 11 to be inserted through the channel while the distal end of the guide catheter 32 remains at the predetermined position, and permits the distal end of the pusher tube 11 to move to a desirable position. The drainage tube 11 is pushed toward the distal end of the 9 to be inserted through the bile duct 7.

Retracting the guide catheter 32, the pusher tube 11, and the guidewire 5 from the bile duct 7 and detaching them from the endoscope 2 eventually allow the drainage tube 11 to be retained in the bile duct 7.

The drainage tube retainer 30 provides the drainage tube 11 and the pusher tube 11 over the guide catheter 32, detached from the cap 31, not only from the distal end but also from the proximal end, thereby a wide selection of manipulation can be obtained.

In addition, the endoscopic adapter 22 attached to the operation section 12 of the endoscope 2 permits the proximal end of the guide catheter 32 to be fixed separately from the operation section 12 of the endoscope 2 by a predetermined distance and to be directed toward the endoscope port 3 similarly to the first embodiment.
The guidewire 5 and the guide catheter 32 can be folded together at some midpoints thereof in the vicinity of the endoscope port 3; therefore, the guidewire 5 can be disposed substantially in parallel with the guide catheter 32. This allows the direction in which the guide catheter 32 is inserted through the channel from the endoscope port 3 to substantially coincide with the direction in which the guidewire 5 is retracted from the guide catheter 32. This furthermore allows the direction in which the pusher tube 11 is inserted through the channel from the endoscope port 3 to substantially coincide with the direction in which the guide catheter 32 is retracted from the pusher tube 11.

This results in allowing the endoscopist alone to conduct the operation of inserting the guide catheter 32 into the channel along the guidewire 5 while the distal end the guidewire 5 is retained at a certain position. Also, the endoscopist alone having detached the cap 31 can conduct the operation of inserting the drainage tube 6 and the pusher tube 11 through the channel along the guidewire 5 and the guide catheter 32 while the distal end of the guidewire 5 and the distal end of the guide catheter 32 are retained at certain positions.

The technical scope of the present invention is not limited to the embodiments described above. Rather, various modifications may be added provided as long as they do not depart from the spirit of the invention.
For example, the drainage tube retainers 1 and 30 according to the above embodiments are used while the endoscopic adapter 22 is attached to the operation section 12 of the endoscope 2. Note that the hook 16 may be attached without using the endoscopic adapter 22, i.e., directly at a predetermined position of the operation section 12 of the endoscope 2.

Also, as illustrated in FIG. 19, the mark 21 of the drainage tube 40 may be printed at the proximal end of the drainage tube 40 disposed nearer to the endoscope 2 when the drainage tube 40 is disposed in the body. As illustrated in FIG. 19, the mark after the retention of the drainage tube 40 can be observed by using the endoscope if the proximal end of the drainage tube 40 is previously protruded.

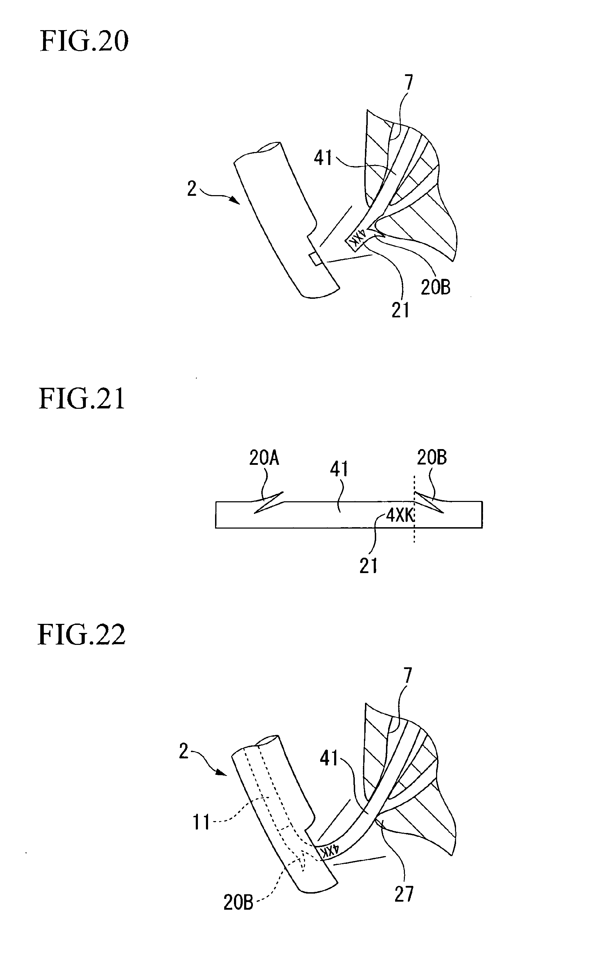

Furthermore, as illustrated in FIG. 21, the mark 21 of the drainage tube 41 may be disposed in the vicinity of the distal end, e.g., a position indicated by a broken line in the drawing, of the flap 20B that is disposed nearer to the endoscope 2 when the drainage tube 40 is retained in the body.

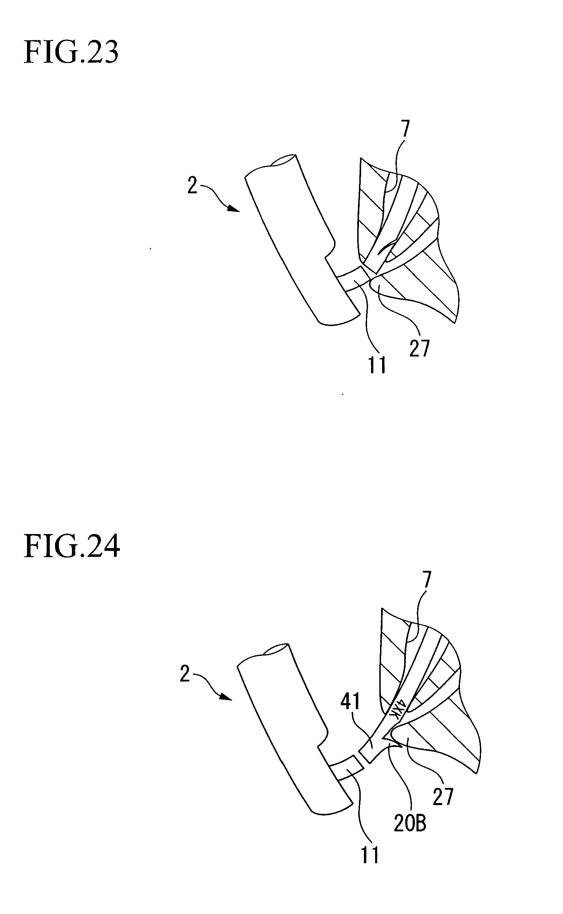

In ordinary cases, the drainage tube 41 is disposed so that a distal end flap 20A expands beyond the constriction 8 of the bile duct 7 and a proximal end flap 20B expands in a duodenum. As illustrated in FIG. 22, the proximal end flap 20B of the drainage tube 41 can be observed when it is extracted from the distal end of the endoscope 2. Accordingly, the proximal end flap is prevented from being inserted into the bile duct 7 due to the excessively extended drainage tube 41 as illustrated in FIG. 23; thus, the drainage tube 41 can be retained at a predetermined position as illustrated in FIG. 24. Industrial Applicability

The present invention relates to an endoscopic treatment instrument inserted through a channel of an endoscope from an endoscope port that includes: an elongated section for slidably supporting a retainer retained in a live organ through the channel; a hollow section disposed in the exterior of the elongated section and slidably supported by the elongated section; and a connecting section provided to the hollow section for positioning the hollow section relative to the endoscope so that a direction in which the hollow section is inserted through the channel substantially coincides with a direction in which the hollow section is retracted from the hollow section. The present invention allows an endoscopist alone to slide a hollow section along an elongated section so that the elongated section that is inserted in the hollow section is retained at a certain position.

## Claims

1. An endoscopic treatment instrument inserted through a channel of an endoscope from an endoscope port, the endoscopic treatment instrument comprising:
an elongated section for slidably supporting a retainer retained in a live organ through the channel;
a hollow section disposed in the exterior of the elongated section and slidably supported by the elongated section; and
a connecting section provided to the hollow section for positioning the hollow section relative to the endoscope so that a direction in which the hollow section is inserted through the channel substantially coincides with a direction in which the hollow section is retracted from the hollow section.

2. The endoscopic treatment instrument according to Claim 1, wherein the elongated section is capable of sliding along a guidewire inserted through the channel.

3. The endoscopic treatment instrument according to one of Claims 1 and 2, wherein the connecting section is provided with an elastically deformable, approximately C-shaped engagement member.

4. The endoscopic treatment instrument according to one of Claims 1 to 3 , wherein the connecting section attached to the endoscope via an endoscope adapter attached to an operation section of the endoscope for rotatively supporting the connecting section is detachable.

5. The endoscopic treatment instrument according to one of Claims 1 to 4 , further comprising a cap disposed at a proximal end of the elongated section, wherein the cap is detachable from the connecting section.

6. The endoscopic treatment instrument according to one of Claims 1 to 4 , further comprising a cap disposed at a proximal end of the elongated section, wherein the cap is detachable from the elongated section.

7. The endoscopic treatment instrument according to one of Claims 5 and 6 , wherein the cap is provided with the elastically deformable, approximately C-shaped engagement member.

8. The endoscopic treatment instrument according to one of Claims 5 to 7 , wherein the cap is detachable from the endoscope via the endoscope adapter attached to the operation section of the endoscope so as to rotatively support the cap.
